Europäisches Patentamt

European Patent Office

Office européen des brevets

(11) Veröffentlichungsnummer: **0 362 655**
**A1**

## (12) EUROPÄISCHE PATENTANMELDUNG

(21) Anmeldenummer: 89117643.0

(22) Anmeldetag: 25.09.89

(51) Int. Cl.⁵: **A61K 9/12 , A61K 47/38 , A61K 47/42**

Patentansprüche für folgenden Vertragsstaat: ES.

(30) Priorität: 01.10.88 DE 3833458

(43) Veröffentlichungstag der Anmeldung:
**11.04.90 Patentblatt 90/15**

(84) Benannte Vertragsstaaten:
**AT BE CH DE ES FR GB IT LI NL**

(71) Anmelder: CASSELLA Aktiengesellschaft
**Hanauer Landstrasse 526**
**D-6000 Frankfurt am Main 61(DE)**

(72) Erfinder: Greve, Rainer, Dr.
**Konrad-Adenauer-Ring 52**
**D-2360 Bad Segeberg(DE)**
Erfinder: Warnke, Giselher, Dr.
**Sägestrasse 8**
**D-7881 Herrischried(DE)**

(74) Vertreter: Urbach, Hans-Georg, Dr. et al
**Hanauer Landstrasse 526**
**D-6000 Frankfurt am Main 61(DE)**

(54) **Verschäumbare arzneiliche Mischungen.**

(57) Verschäumbare arzneiliche Mischung, die Wasser, einen Gel- bzw. Solbildner, eine wirksame Menge eines Arzneimittels sowie ein Treibgas enthält.

EP 0 362 655 A1

EP 0 362 655 A1

## Verschäumbare arzneiliche Mischungen

Die vorliegende Erfindung betrifft verschäumbare arzneiliche Mischungen, die Wasser, einen Gel- bzw. Solbildner, eine wirksame Menge eines Arzneimittels sowie ein Treibgas enthalten.

Das entscheidende Hindernis gegen eine freiwillige und mühelose Einnahme vieler Arzneimittel ist deren schlechter Geschmack. Des weiteren ist es für viele Patienten sehr schwer oder gar unmöglich, größere Tabletten oder Kapseln zu schlucken.

Es ist bereits bekannt, daß diese Schwierigkeiten umgangen werden, wenn das Arzneimittel in einer Form vorliegt, die mit Hilfe eines Treibgases verschäumt werden kann. Vorteilhafterweise ist eine solche Mischung in einem druckfesten, mit einem Dosierventil ausgestatteten Behältnis enthalten, dem auf bequeme Art und Weise eine genau dosierte Menge eines wohlschmeckenden und leicht schluckbaren Schaumes entnommen werden kann.

Die bisher bekannten Mischungen sind allerdings mit einigen schwerwiegenden Nachteilen behaftet. So beschreibt die internationale Patentanmeldung WO 86/05392 eine verschäumbare wasserfreie Mischung, die hauptsächlich aus fetten Ölen und anderen Fettsäureestern besteht. Das Arzneimittel liegt in dieser Mischung in einer öligen Phase vor und muß vor der Resorption im Magen-Darm-Trakt erst daraus freigesetzt werden, ist also relativ schlecht bioverfügbar.

Weiterhin stellen die fetten Bestandteile eine nicht unerhebliche Kalorienzufuhr dar und können bei empfindli chen Patienten zu Störungen im Leber-Gallen-Bereich führen. Dies trifft auch auf die verschäumbaren Öl-in-Wasser-Emulsionen zu, die in der GB 1,121,358 beschrieben sind.

Aufgabe vorliegender Erfindung ist es deshalb, eine verschäumbare arzneiliche Mischung bereitzustellen, die den Organismus des Patienten nicht belastet und die sich durch eine hohe Bioverfügbarkeit der Wirkstoffe auszeichnet.

Diese Aufgabe wird in überraschender Weise hervorragend gelöst durch eine verschäumbare arzneiliche Mischung, die dadurch gekennzeichnet ist, daß sie Wasser, einen Gel-bzw. Solbildner, eine wirksame Menge eines Arzneimittels sowie ein Treibgas enthält.

Als Gel- bzw. Solbildner kommen bevorzugt wasserlösliche Cellulosederivate oder Gelatine zur Anwendung. Ein besonders bevorzugtes Cellulosederivat ist Methylcellulose. Die Gel- bzw. Solbildner werden bevorzugt in Mengen von 0,1 bis 10 Gew.%, besonders bevorzugt 1 bis 4 Gew.%, eingesetzt.

Als Treibgas kommen alle toxikologisch unbedenklichen Gase in Frage. Bevorzugt sind Kohlendioxid und Distickstoffmonoxid (Lachgas). Die Mengen, in denen die Treibgase eingesetzt werden, sind vorteilhafterweise so bemessen, daß sie gerade zur vollständigen Verschäumung der Mischung ausreichen. Besonders bevorzugt werden sie in Mengen von 1 bis 4 Gew.% eingesetzt.

Im Prinzip können die erfindungsgemäßen Mischungen alle Arzneimittel enthalten, die oral verabreicht werden können. Diese können synthetisch hergestellt oder Natur stoffe, wasserlöslich oder wasserunlöslich sein. Auch Mischungen verschiedener Arzneimittel können eingesetzt werden. Bevorzugt sind pflanzliche Extrakte und wasserlösliche Wirkstoffe, die besonders bevorzugt in Form von Konzentraten oder Sirupen eingesetzt werden.

Die dabei verwendeten Mengen liegen bevorzugt zwischen 10 und 90 Gew.%, besonders bevorzugt zwischen 30 und 80 Gew.%.

Das in den erfindungsgemäßen Mischungen enthaltene Wasser ist für pharmazeutische Zwecke geeignetes, gereinigtes Wasser. Die eingesetzten Mengen ergänzen die Mischungen zu 100 Gew.%.

Die erfindungsgemäßen Mischungen können gegebenenfalls auch übliche Hilfsmittel, beispielsweise Stabilisierungs-, Konservierungs-, Süß-, Färbe-, Geschmacks- oder Aromatisierungsmittel, enthalten.

Die erfindungsgemäßen Mischungen sind bevorzugt in einem druckfesten Behältnis enthalten, das mit einem Dosierventil ausgerüstet ist.

Die erfindungsgemäßen Mischungen können hergestellt werden, indem zuerst der Sol- oder Gelbildner mit dem Wasser vorgequollen und in ein druckfestes Behältnis gefüllt, anschließend das Arzneimittel zugegeben und das Behältnis mit einem Ventil verschlossen und zuletzt das Treibgas über das Ventil eingefüllt wird.

Durch Betätigen des Dosierventils kann die erfindungsgemäße Mischung zu einem arzneilichen Schaum verschäumt werden, der gut schmeckt und der problemlos geschluckt werden kann. Die darin enthaltenen Arzneistoffe sind in der Wasserphase gelöst oder suspendiert, was eine hervorragende Bioverfügbarkeit bewirkt.

Der Schaum enthält keinerlei Öle oder zusätzliche Schaumbildner, also keine Emulgatoren, Tenside, Saponine oder ähnliche Verbindungen und ist somit leicht verträglich und physiologisch unbedenklich. Ein weiterer Vorteil besteht darin, daß auf umweltschädigende Treibgase der Erdölfraktion (z.B. Kohlenwasser-

2

stoffe) oder halogenierte Kohlenwasserstoffe verzichtet werden kann.

Die folgenden Beispiele 1 bis 6 zeigen Ausführungsmöglichkeiten vorliegender Erfindung.

| Beispiel 1 | |
|---|---|
| Mischung gegen Husten | |
| 1,5 g | Weiße Gelatine |
| 36,0 g | Gereinigtes Wasser |
| 60,0 g | Hustensaft-Konzentrat |
| 2,5 g | Kohlendioxid |
| 100,0 g | |

| Beispiel 2 | |
|---|---|
| Mischung gegen Husten | |
| 2,0 g | Methylcellulose |
| 49,0 g | Gereinigtes Wasser |
| 45,0 g | Hustentropfen-Konzentrat |
| 4,0 g | Distickstoffmonoxid |
| 100,0 g | |

| Beispiel 3 | |
|---|---|
| Mischung zum Abführen | |
| 3,0 g | Methylcellulose |
| 58,0 g | Gereinigtes Wasser |
| 35,0 g | Sennosid-Sirup |
| 4,0 g | Distickstoffmonoxid |
| 100,0 g | |

| Beispiel 4 | |
|---|---|
| Vitamin-Mischung | |
| 2,5 g | Weiße Gelatine |
| 40,0 g | Gereinigtes Wasser |
| 54,0 g | Multivitamin-Konzentrat-Sirup |
| 3,5 g | Kohlendioxid |
| 100,0 g | |

| Beispiel 5 | |
|---|---|
| Mischung gegen Durchfall | |
| 3,5 g | Methylcellulose |
| 45,0 g | Gereinigtes Wasser |
| 49,0 g | Tannin-Eiweiß-Suspension |
| 2,5 g | Kohlendioxid |
| 100,0 g | |

| Beispiel 6 | |
|---|---|
| Beruhigungsmittel für Kinder | |
| 1,0 g | Weiße Gelatine |
| 20,0 g | Gereinigtes Wasser |
| 78,0 g | gesüßtes Baldriankonzentrat |
| 1,0 g | Kohlendioxid |
| 100,0 g | |

## Ansprüche

1. Verschäumbare arzneiliche Mischungen, dadurch gekennzeichnet, daß sie Wasser, einen Gel- bzw. Solbildner, eine wirksame Menge eines Arzneimittels sowie ein Treibgas enthalten.

2. Mischung gemäß Anspruch 1, dadurch gekennzeichnet, daß als Gel- bzw. Solbildner wasserlösliche Cellulosederivate oder Gelatine enthalten sind.

3. Mischung gemäß Anspruch 2, dadurch gekennzeichnet, daß als wasserlösliches Cellulosederivat Methylcellulose enthalten ist.

4. Mischung gemäß einem oder mehreren der Ansprüche 1 bis 3, dadurch gekennzeichnet, daß als Treibgas Kohlendioxid oder Distickstoffmonoxid enthalten ist.

5. Mischung gemäß einem oder mehreren der Ansprüche 1 bis 4, dadurch gekennzeichnet, daß sie in einem druckfesten, mit einem Dosierventil ausgestatteten Behältnis enthalten ist.

6. Verfahren zur Herstellung der verschäumten arzneilichen Mischung gemäß Ansprüchen 1 bis 4, dadurch gekennzeichnet, daß zuerst der Sol- oder Gelbildner mit dem Wasser vorgequollen und in ein druckfestes Behältnis gefüllt, anschließend das Arzneimittel zugegeben und das Behältnis mit einem Ventil verschlossen und zuletzt das Treibgas über das Ventil eingefüllt wird.

Patentansprüche für folgenden Vertragsstaat: ES

1. Verfahren zur Herstellung einer verschäumbaren arzneilichen Mischung, enthaltend Wasser, einen Gel- bzw. Solbildner, eine wirksame Mange eines Arzneimittels sowie ein Treibgas, dadurch gekennzeichnet, daß zuerst der Sol-oder Gelbildner mit dem Wasser vorgequollen und in ein druckfestes Behältnis gefüllt, anschließend das Arzneimittel zugegeben und das Behältnis mit einem Ventil verschlossen und zuletzt das Treibgas über das Ventil eingefüllt wird.

2. Verfahren gemäß Anspruch 1, dadurch gekennzeichnet, daß als Gel- bzw. Solbildner wasserlösliche Cellulosederivate oder Gelatine eingesetzt wird.

3. Verfahren gemäß Anspruch 2, dadurch gekennzeichnet, daß als wasserlösliches Cellulosederivat Methylcellulose eingesetzt wird.

4. Verfahren gemäß einem oder mehreren der Ansprüche 1 bis 3, dadurch gekennzeichnet, daß als Treibgas Kohlendioxid oder Distickstoffmonoxid eingesetzt wird.

5. Verfahren gemäß einem oder mehreren der Ansprüche 1 bis 4, dadurch gekennzeichnet, daß das Behältnis mit einem Dosiermittel verschlossen wird.

4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 801 863 (UPJOHN CO.)<br>* Ansprüche 1,2 *<br>--- | 1-3 | A 61 K 9/12<br>A 61 K 47/38<br>A 61 K 47/42 |
| X | US-A-3 051 621 (D.M. GREEN et al.)<br>* Spalte 2, Zeile 31 - Spalte 3, Zeile 13; Ansprüche *<br>--- | 1-6 | |
| X | US-A-3 144 386 (G.E. BRIGHTENBACK)<br>* Spalte 1, Zeilen 23-39 *<br>----- | 1,5 | |

RECHERCHIERTE SACHGEBIETE (Int. Cl.5)

A 61 K 9/00
A 61 K 47/00

Der vorliegende Recherchenbericht wurde für alle Patentansprüche erstellt

| Recherchenort | Abschlußdatum der Recherche | Prüfer |
|---|---|---|
| DEN HAAG | 05-12-1989 | SITCH W.D.C. |